# FASCICULE DE BREVET EUROPEEN

(11) **EP 0 732 115 B1**
(45) Date de publication et mention de la délivrance du brevet: **05.06.2002**
(21) Numéro de dépôt: 96400498.0
(22) Date de dépôt: 11.03.1996
(51) Int. Cl.: A61M 5/315

(54) **Appareil pour l'injection d'un liquide**
Vorrichtung zur Injektion einer Flüssigkeit
Device for injecting a liquid

(30) Priorité: 13.03.1995 FR 9502874
(43) Date de publication de la demande: 18.09.1996
(73) Titulaire: VYGON, F-95440 Ecouen (FR)
(72) Inventeur: Brinon, Thierry, 95560 Montsoult (FR)
(74) Mandataire: Schrimpf, Robert

(56) Documents cités:
- EP-A- 0 483 759
- WO-A-93/24160
- DE-U- 9 107 574

## Description

L'invention concerne un appareil portable et non implantable pour permettre à un patient de s'administrer, à sa demande, des doses successives d'un liquide sans que le délai entre deux administrations successives puisse être inférieur à une valeur de consigne.

On connait des appareils tels que définis ci-dessus qui comprennent :
- une chambre munie d'une sortie d'injection ;
- un moyen anti-retour coopérant avec ladite sortie ;
- un piston rigide apte à être poussé dans la chambre en direction de ladite sortie, avec étanchéité latérale autour du piston, et à revenir en sens inverse sous l'action d'un moyen de rappel;
- un moyen limitant la course du piston en sens inverse en sorte qu'en fin de course de rappel du piston, le volume de la chambre corresponde au volume d'une dose à administrer ;
- et un réservoir de liquide ayant une capacité de plusieurs doses et communiquant avec la chambre par l'intermédiaire d'un limiteur de débit correspondant à la valeur de consigne.

La publication EP-0483759 décrit un tel appareil.

Dans cette réalisation, le réservoir et la pompe sont séparés l'un de l'autre et ne peuvent être manipulés ensemble d'une seule main.

La publication DE-U-9107574 décrit un appareil de type seringue pour administrer à la demande une dose d'un produit liquide ou pâteux, en particulier dans le domaine dentaire, qui comporte un carter tubulaire allongé dont une extrémité est adaptée pour le montage d'un réservoir, lequel contient plusieurs doses du produit, à une extrémité d'un piston creux qui se déplace dans le carter sur une course limitée, sous la commande d'une manette latérale, et qui est rappelé par un ressort, l'extrémité opposée du carter contenant une chambre de dosage dans laquelle se déplace l'autre extrémité du piston avec étanchéité latérale et qui communique avec un canal de sortie du carter par l'intermédiaire d'un moyen anti-retour.

Cet appareil est plus compact que celui de la publication EP-0483759 mais le passage du réservoir à la chambre est contrôlé par un jeu de valves qui fonctionne par tout ou rien et l'appareil n'est pas conçu pour que le délai entre deux administrations successives soit inférieur à une valeur de consigne.

La présente invention a pour but de fournir un appareil compact, facile à manipuler, réduisant les risques d'incidents, pour permettre à un patient de s'administrer, à sa demande, des doses successives d'un liquide sans que le délai entre deux administrations successives puisse être inférieur à une valeur de consigne.

On y parvient selon l'invention avec un appareil tel que défini dans la revendication 1.

De préférence, l'appareil est monobloc, tous les éléments de l'appareil étant contenus dans un carter tubulaire fermé par un couvercle laissant accessible un poussoir solidaire du piston. L'appareil ne comporte aucune tubulure extérieure qui pourrait constituer une cause d'accident, à l'exception de la tubulure raccordée à la sortie d'injection.

Le carter sert à guider le piston, lequel peut coulisser dans le carter avec ou sans jeu latéral.

On décrira ci-après, à titre d'exemple non limitatif, un tel mode de réalisation, la description et les figures faisant apparaître d' autres particularités intéressantes qui sont réunies dans cette réalisation mais qui, dans des variantes de réalisation, peuvent être utilisées individuellement ou selon des combinaisons partielles.

Sur les figures :
- la fig. 1 est une coupe axiale de l'appareil. le piston en position haute ;
- la fig. 2 est une vue partielle similaire, le piston étant en position basse
- la fig. 3 est une vue agrandie de l'extrémité inférieure de la fig. 1,
- la fig. 4 est une coupe axiale de l'appareil dont on a ôté la partie du piston qui forme réservoir et qui est démontable, ainsi que le couvercle du carter de l'appareil
- la fig. 5 est une vue extérieure du fond du carter ;
- la fig. 6 est une coupe axiale de la partie du piston qui forme réservoir et qui peut être dissociée du reste du piston ;
- la fig. 7 est une vue en coupe du couvercle du carter de l'appareil, et
- la fig. 8 est une coupe axiale d'une variante de l'appareil.

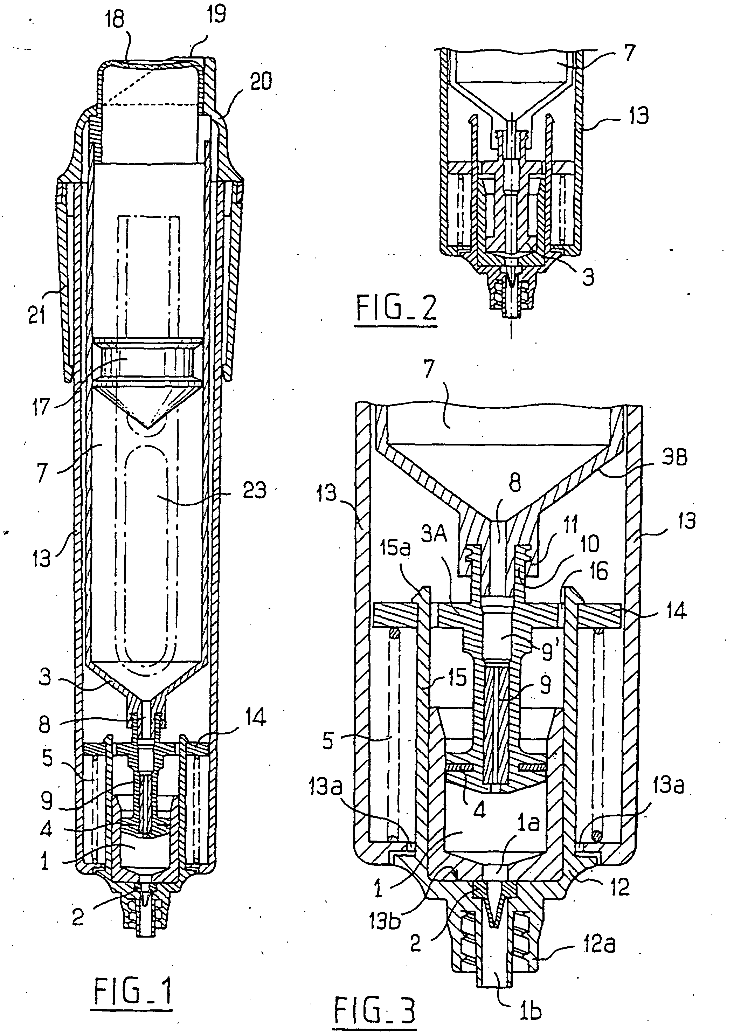

L'appareil représenté sur les figures est un appareil monobloc qui comprend, dans un carter cylindrique 13 :
- une chambre 1 munie d'une sortie d'injection 1a ;
- un moyen anti-retour 2, par exemple un clapet anti-retour en soi connu, coopérant avec la sortie la pour empêcher qu'après une injection du liquide injecté soit réaspiré dans la chambre par cette sortie lors de la course de rappel du piston ;
- un piston rigide 3 apte à être poussé axialement dans la chambre en direction de la sortie 1a, avec étanchéité latérale 4 autour du piston, et à revenir en sens inverse sous l'action d'un moyen de rappel, en sorte qu'en fin de course de rappel (position représentée sur la figure 1), le volume V de la chambre corresponde au volume d'une dose à administrer;
- un réservoir de liquide 7 formé dans le piston et communiquant avec l'intérieur de la chambre 1 par un passage 8 formé dans le piston et muni d'un tube capillaire 9 choisi en fonction de la valeur de consigne.

Avantageusement le passage 8 qui contient le tube capillaire 9 comporte également un filtre 9' en amont du capillaire.

Avantageusement, le piston 3 est constitué d' une partie de piston inférieure 3A qui pénètre dans la chambre 1 avec étanchéité latérale 4 et d' une partie de piston supérieure 3B qui constitue une cartouche contenant ledit réservoir 7 et qui, de préférence, est fixée à la partie inférieure par des moyens permettant son montage et son démontage, ce qui est le cas de l'exemple représenté.

Ces moyens, dans l'exemple représenté, sont un ajutage conique femelle 10 formé à l'extrémité haute de la partie de piston inférieure 3A et dans lequel pénètre un nez mâle 11 formé à l'extrémité basse de la partie de piston supérieure 3B, l'ajutage et le nez présentant des filetages coopérant pour assurer cette fixation par vissage. (fig. 3).

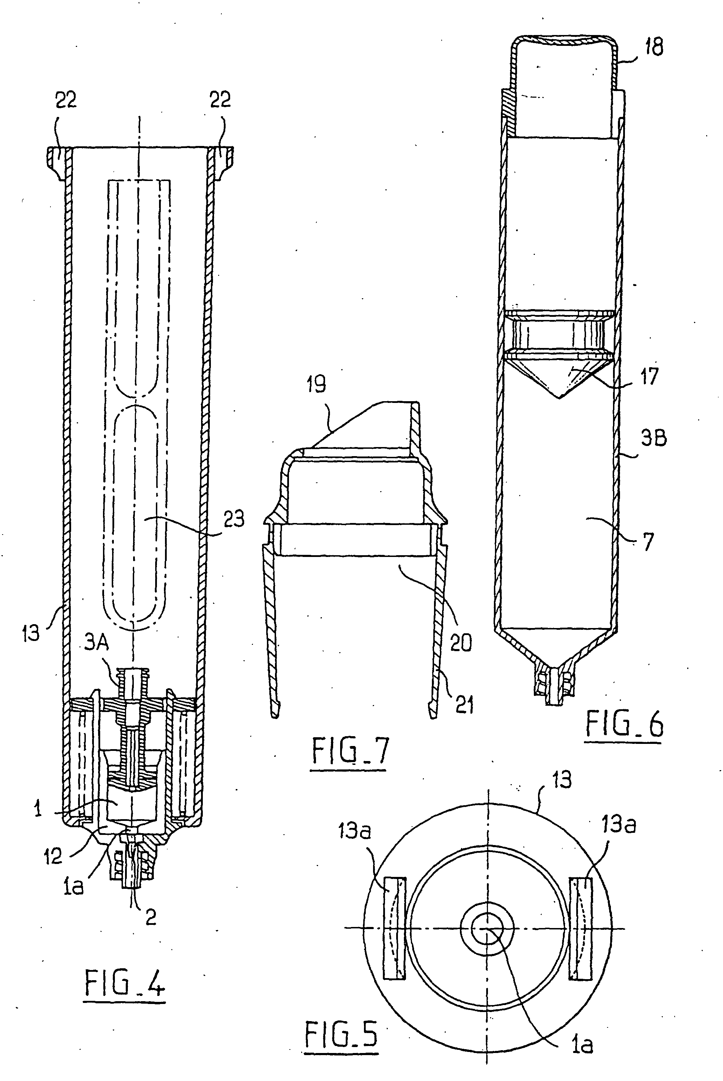

Dans cette réalisation, la chambre 1 vient de moulage avec le boîtier 13 et le fond du carter boîtier présente, de part et d'autre de la chambre, deux fenêtres 13a (fig. 5) dans lesquelles passent deux pattes 15 solidaires d'une pince 12 qui est rapportée contre la face extérieure 13b du fond du boîtier pour maintenir le moyen anti-retour 2 contre la sortie 1a (fig. 3)et qui présente un ajutage de sortie 12a pour le branchement d'une tubulure, de façon en soi connue.

Dans cette réalisation ladite pièce rapportée et le piston comportent des moyens qui coopèrent pour limiter la course de rappel du piston.

Par exemple, le piston 3 comporte une traverse 14 qui, lorsque le piston se déplace, coulisse le long des pattes 15. En fin de course de rappel le piston vient buter contre les extrémités 15a des pattes, ce qui détermine la fin de cette course.

Les perçages 16 de la traverse 14 sont suffisamment larges pour laisser passer les têtes 15a des pattes lorsque le piston est mis en place dans le carter avec un pincement momentané des pattes.

Le ressort de rappel 5 est avantageusement placé dans le carter autour de la pièce rapportée 12 et prend appui sur la traverse.

La traverse peut avoir tout forme désirée, par exemple la forme d'un disque.

Comme on le voit sur la fig. 1, le réservoir 7 contient un bouchon 17 qui suit le niveau du liquide dans le réservoir en empêchant tout volume mort au dessus du liquide dans le réservoir et en garantissant l'étanchéité de l'appareil dans toutes les positions.

Le piston 3 est fermé à son extrémité supérieure par un bouchon poussoir 18 qui fait saillie hors d'une ouverture 19 formée dans un couvercle 20 monté à l'extrémité supérieure du carter 13. Dans la réalisation représentée sur les figs. 1 à 7, ce couvercle 20 comporte des pattes 21 qui sont poussées vers le bas dans des passages 22 formés sur le côté du carter jusqu'à ce que les extrémités supérieures des pattes soient coincées dans ces passages, en sorte que pour retirer le couvercle il soit nécessaire de casser ces pattes. Il n'est donc pas possible de retirer ou de remplacer subrepticement la cartouche 3B.

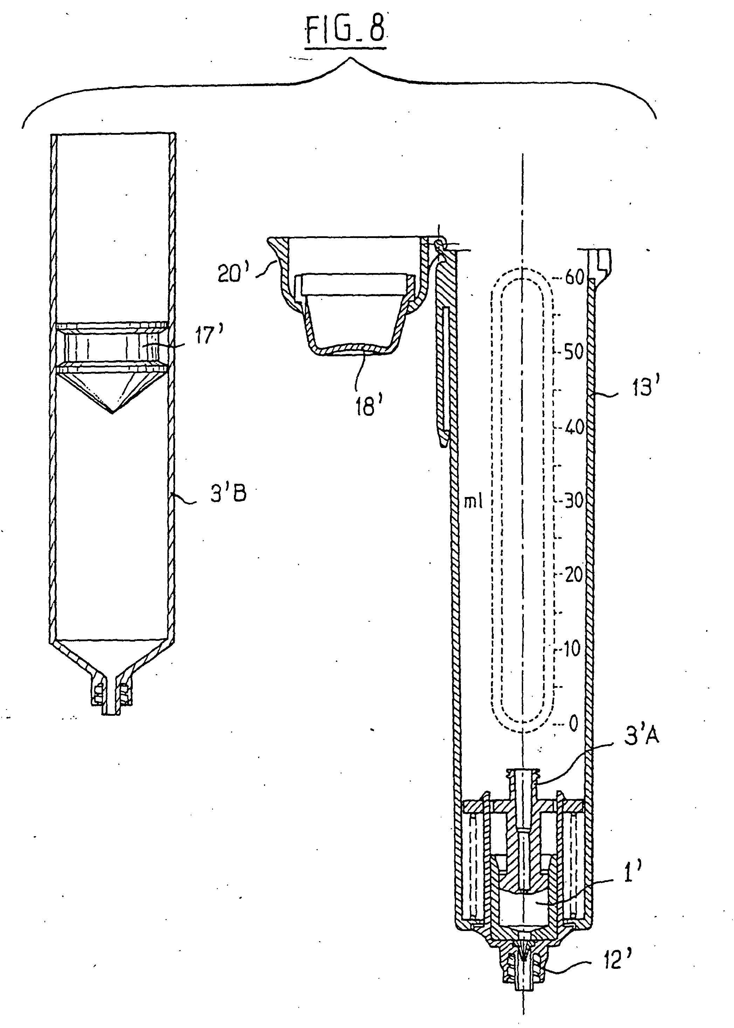

En variante, le couvercle est monté de façon à pouvoir être ouvert, comme dans la réalisation de la fig. 8, montrée à titre d'exemple, dont le couvercle 2o' est monté à pivotement sur le carter 13'.

Le bouchon poussoir 18 est percé pour permettre la mise à l'air du réservoir au dessus du bouchon mobile en sorte que la pression atmosphérique s'exerce sur le bouchon mobile.

Avantageusement, le carter présente des parties transparentes ou percées 23, munies éventuellement de repères, qui permettent d'observer le bouchon 17, et de connaître par conséquent le volume de liquide résiduel.

L'appareil est susceptible de nombreuses variantes. Par exemple, dans une réalisation avantageuse, le carter de l'appareil est constitué de parties séparables en sorte que la zone de fixation des deux parties 3A, 3B du piston soit accessible après démontage du carter, ce qui facilite toute intervention au niveau de cette zone, notamment au moment d'un changement de la cartouche de réservoir 3B.

Il est également prévu, dans une variante, que la chambre, au lieu d'être solidaire du carter, soit portée par la pièce rapportée, le carter présentant alors un fond suffisamment ouvert pour l'introduction de la chambre par ce fond.

### FONCTIONNEMENT

La figure 1 représente l'appareil prêt à l'emploi. A partir de la position du piston représentée sur la fig. 1, le patient pousse manuellement le piston jusqu'à ce que ce dernier arrive en fin de course au fond de la chambre 1 contre la sortie 1a, cette chambre est ainsi purgée (fig. 2).

Après relâchement du bouchon poussoir 18, le piston revient quasi immédiatement à sa position initiale (figure 1) grâce au ressort de rappel (5).

La chambre 1 est ainsi mise en dépression puisque la valve anti-retour 2 de type "bec de canard" empêche toute entrée dans la chambre par la sortie de la chambre.

Cette dépression provoque une aspiration par le passage 8 du liquide contenu dans le réservoir 7 jusqu'à remplissage de la chambre, la durée du remplissage étant déterminée par les dimensions du capillaire placé dans ce passage.

## Revendications

1. Appareil portable et non implantable pour permettre à un patient de s'administrer, à sa demande, des doses successives d'un liquide sans que le délai entre deux administrations successives puisse être inférieur à une valeur de consigne, cet appareil comprenant :
- une chambre (1) munie d'une sortie d'injection (1a) :
- un moyen anti-retour (2) coopérant avec ladite sortie ;
- un piston rigide (3) apte à être poussé dans la chambre en direction de ladite sortie, avec étanchéité latérale (4) autour du piston, et à revenir en sens inverse sous l'action d'un moyen de rappel (5) ;
- un moyen (15a) limitant la course du piston en sens inverse en sorte qu'en fin de course de rappel du piston, le volume (1) de la chambre (1) corresponde au volume d'une dose à administrer ;
- un réservoir de liquide ayant une capacité de plusieurs doses et communiquant avec la chambre par l'intermédiaire d'un limiteur de débit (9) ;
**caractérisé en ce que** le réservoir de liquide (7) est formé dans le piston (3) et communique avec l'intérieur de la chambre (1) par un passage (8) formé dans le piston (3) et muni du dit limiteur de débit (9) tel que la durée de remplissage de la chambre, par aspiration du liquide du réservoir sous l'effet de la dépression causée dans la chambre par le rappel du piston, corresponde à la valeur de consigne.

2. Appareil selon la revendication 1, **caractérisé en ce que** le piston (3) comporte une partie de piston inférieure (3A) qui pénètre dans la chambre (1) avec étanchéité latérale (4) et une partie de piston supérieure (3B) qui contient ledit réservoir (7) et qui est fixée à la partie inférieure par des moyens (10,11) permettant son montage et son démontage.

3. Appareil selon la revendication 1 ou 2, **caractérisé en ce qu'**il comporte un carter tubulaire (13) qui contient le piston (3) et la chambre (1), en laissant l'accès à un poussoir (18) solidaire du piston.

4. Appareil selon la revendication 3, **caractérisé** ence qu'il ne comporte aucune tubulure extérieure au dit carter sauf celle qui est raccordée à la sortie d'injection.

5. Appareil selon l'une des revendications 3 et 4, **caractérisé en ce qu'**il comporte une pièce (12) rapportée de l'extérieur contre le fond du carter tubulaire (13) pour maintenir le moyen anti-retour (2) et pour fournir un ajutage 12a de raccordement d'une tubulure.

6. Appareil selon la revendication 5, **caractérisé en ce que** ladite pièce rapportée (12) présente des pattes (15) qui pénètrent dans le carter par des fentes (13a) formées dans le fond du carter et qui agissent pour limiter la course de rappel du piston.

7. Appareil selon la revendication 6, **caractérisé en ce que** le piston (3) comporte une traverse (14) qui coulisse le long des pattes (15) lorsque le piston est poussé et qui vient buter contre les extrémités (15a) des pattes lorsque le piston est rappelé en sens inverse, ce qui détermine la fin de course de rappel du piston.

8. Appareil selon la revendication 7, **caractérisé en ce que** le moyen de rappel est un ressort (5) qui prend appui sur ladite traverse (14).

9. Appareil selon la revendication 2 et l'une des revendications 3 à 8 **caractérisé en ce que** ledit carter (13) est constitué de parties séparables pour rendre accessible la zone de fixation de la partie de piston supérieure (3B) à la partie de piston inférieure (3A).

10. Appareil selon l'une des revendications 2 à 9, **caractérisé en ce que** ledit carter (13) est fermé à son extrémité opposée à la chambre par un couvercle (20) qui autorise une poussée manuelle sur le piston mais qui interdit, sauf à être cassé, d'extraire du carter la partie du piston qui comporte ledit réservoir (7) .

11. Appareil selon l'une des revendications 1 à 10, **caractérisé en ce que** la partie du piston (3) qui constitue le réservoir (7) contient un bouchon (17) qui suit le niveau du liquide dans le réservoir en empêchant tout volume mort au dessus du liquide dans le réservoir et en garantissant l'étanchéité de l'appareil dans toutes les positions.

12. Appareil selon la revendication 11, **caractérisé en ce qu'**il présente des parties transparentes ou percées en sorte que le bouchon (17) soit visible de l'extérieur de l'appareil de façon à permettre de connaître le volume résiduel de liquide dans le réservoir.

13. Appareil selon l'une des revendications 1 à 12, **caractérisé en ce que** ledit limiteur de débit (9) est un tube capillaire choisi en fonction de la valeur de consigne.

## Patentansprüche

1. Tragbares und nicht-implantierbares Gerät, um es einem Patienten zu gestatten, sich nach Wunsch aufeinanderfolgende Dosen einer Flüssigkeit zu verabreichen, ohne daß die zeitliche Verzögerung zwischen zwei aufeinanderfolgenden Verabreichungen kleiner sein kann als ein Soll- bzw. Einstellwert, mit:
- einer Kammer (1), die mit einem Injektionsaustritt (1a) versehen ist;
- einer Rückschlageinrichtung (2), die mit dem genannten Austritt zusammenwirkt;
- einem starren Kolben (3), der dazu eingerichtet ist, in der Kammer in Richtung des genannten Austritts verschoben zu werden, mit einer seitlichen Abdichtung (4) rund um den Kolben, und in umgekehrter Richtung unter Wirkung eines Rückstellmittels (5) zurückzukehren;
- einem Mittel (15a), das den Weg des Kolbens in umgekehrter Richtung so begrenzt, daß am Ende des Rückstellweges des Kolbens das Volumen (1) der Kammer (1) dem Volumen einer zu verabreichenden Dosis entspricht; und
- einem Flüssigkeits-Vorratsbehälter, der ein Fassungsvermögen mehrerer Dosen aufweist und mit der Kammer mittels eines Durchsatzbegrenzers (9) in Verbindung steht;
**dadurch gekennzeichnet, daß** der Flüssigkeits-Vorratsbehälter (7) im Kolben (3) ausgebildet ist und mit dem Innenraum der Kammer (1) durch einen Durchlaß (8) im Verbindung steht, der im Kolben (3) ausgebildet ist und mit dem genannten Durchsatzbegrenzer (9) versehen ist, so daß die Nachfülldauer der Kammer durch Ansaugen der Flüssigkeit des Vorratsbehälters unter Wirkung des Unterdrucks, der in der Kammer durch die Rückstellung des Kolbens verursacht ist, dem Einstellwert entspricht.

2. Gerät nach Anspruch 1, **dadurch gekennzeichnet, daß** der Kolben (3) einen unteren Kolbenteil (3A) aufweist, der in die Kammer (1) mit seitlicher Abdichtung (4) eindringt, sowie einen oberen Kolbenteil (3B), der den genannten Vorratsbehälter (7) enthält und der am unteren Teil durch Mittel (10, 11) befestigt ist, die seine Montage und Demontage gestatten.

3. Gerät nach Anspruch 1 oder 2, **dadurch gekennzeichnet, daß** es ein rohrförmiges Gehäuse (13) enthält, das den Kolben (3) und die Kammer (1) enthält, indem es einen Zugang für einen Stößel (18) beläßt, der mit dem Kolben ein festes Teil bildet.

4. Gerät nach Anspruch 3, **dadurch gekennzeichnet, daß** es keinerlei Rohrleitung außerhalb des genannten Gehäuses außer der aufweist, die mit dem Injektionsaustritt verbunden ist.

5. Gerät nach einem der Ansprüche 3 und 4, **dadurch gekennzeichnet, daß** es ein Teil (12) aufweist, das von außen her gegen den Boden des rohrförmigen Gehäuses (13) angesetzt ist, um das Rückschlagmittel (2) zu halten und einen Ansatz (12a) zum Anfügen einer Rohrleitung anzufügen.

6. Gerät nach Anspruch 5, **dadurch gekennzeichnet, daß** das genannte angesetzte Teil (12) Klauen (15) aufweist, die in das Gehäuse durch Schlitze (13a) eindringen, die im Boden des Gehäuses ausgebildet sind und die es bewirken, den Rückstellweg des Kolbens zu begrenzen.

7. Gerät nach Anspruch 6, **dadurch gekennzeichnet, daß** der Kolben (3) einen Querträger (14) aufweist, der längs der Klauen (15) verschieblich ist, wenn der Kolben verschoben wird, und der zum Anschlag gegen die Enden (15a) der Klauen gelangt, wenn der Kolben in umgekehrter Richtung zurückgestellt wird, was das Ende des Rückstellweges des Kolbens bestimmt.

8. Gerät nach Anspruch 7, **dadurch gekennzeichnet, daß** das Rückstellmittel eine Feder (5) ist, die sich auf dem genannten Querträger (14) abstützt.

9. Gerät nach Anspruch 2 und einem der Ansprüche 3 bis 8, **dadurch gekennzeichnet, daß** das genannte Gehäuse (13) aus Teilen gebildet ist, die trennbar sind, um die Zone der Befestigung des oberen Kolbenteils (3B) am unteren Kolbenteil (3A) zugänglich zu machen.

10. Gerät nach einem der Ansprüche 2 bis 9, **dadurch gekennzeichnet, daß** das genannte Gehäuse (13) an seinem Ende, das der Kammer gegenüberliegt, durch einen Deckel (20) verschlossen ist, der einen Druck von Hand auf den Kolben ermöglicht, aber es, soweit er nicht zu Bruch geht, untersagt, den Teil des Kolbens aus dem Gehäuse zu ziehen, der den genannten Vorratsbehälter (7) aufweist.

11. Gerät nach einem der Ansprüche 1 bis 10, **dadurch gekennzeichnet, daß** der Teil des Kolbens (3), der den Vorratsbehälter (7) bildet, einen Stopfen (17) enthält; der dem Flüssigkeitsspiegel im Vorratsbehälter nachfolgt, dabei jedes freie Volumen über der Flüssigkeit im Vorratsbehälter verhindert und dabei die Abdichtung des Gerätes in allen Lagen garantiert.

12. Gerät nach Anspruch 11, **dadurch gekennzeichnet, daß** es durchsichtige oder durchlochte Teile derart aufweist, daß der Stopfen (17) von der Außenseite des Gerätes her sichtbar ist, um es auf dieses Weise zu gestatten, daß man das Restvolumen an Flüssigkeit im Vorratsbehälter erkennt.

13. Gerät nach einem der Ansprüche 1 bis 12, **dadurch gekennzeichnet, daß** der genannte Durchsatzbegrenzer (9) ein Kapillarrohr ist, das in Abhängigkeit vom Einstellwert gewählt ist.

## Claims

1. A portable and non-implantable device for enabling a patient to self-administer, on demand, successive doses of a liquid while ensuring that the time period between two successive administrations cannot be less than a reference value, the device comprising:
a chamber (1) provided with an injection outlet (Ia);
non-return means (2) co-operating with said outlet;
a rigid piston (3) suitable for being pushed inside the chamber towards said outlet, with lateral sealing (4) around the piston, and suitable for returning in the opposite direction under drive from return means (5);
means (15a) limiting the stroke of the piston in the reverse direction so that the volume (V) of the chamber (1) at the end of the piston return stroke corresponds to the volume of a dose to be administered; and
a liquid reservoir having a capacity of several doses and communicating with the chamber via a flow rate limiter (9);
**characterized in that** the liquid reservoir (7) is formed in the piston (3) and communicates with the inside of the chamber (1) via a passage (8) formed through the piston (3) and provided with said flow rate limiter (9) such that the filling time of the chamber by liquid being sucked from the reservoir under the effect of the suction caused in the chamber by return of the piston, corresponds to the reference value.

2. A device according to claim 1, **characterized in that** the piston (3) includes a bottom piston portion (3A) which penetrates into the chamber (1) with lateral sealing (4) and a top piston portion (3B) which contains said reservoir (7) and which is fixed to the bottom portion by means (10, 11) enabling engagement and disengagement.

3. A device according to claim 1 or 2, **characterized in that** it includes a tubular case (13) containing the piston (3) and the chamber (1), leaving access to a pusher (18) secured to the piston.

4. A device according to claim 3, **characterized in that** it includes no external tube outside said case other than the tube connected to its injection outlet.

5. A device according to claim 3 or 4, **characterized in that** it includes a piece (12) applied externally against the bottom of the tubular case (13) to hold the non- return means (2) and to provide a socket (12a) for coupling to a tube.

6. A device according to claim 5, **characterized in that** said externally-applied piece (12) has arms (15) which penetrate into the case via windows (13a) formed in the bottom of the case, which arms act to limit the return stroke of the piston.

7. A device according to claim 6, **characterized in that** the piston (3) includes a cross-member (14) which slides along the arms (15) when the piston is pushed, and which comes into abutment against the ends (15a) of the arms when the piston is returned in the opposite direction, thereby determining the end of the piston return stroke.

8. A device according to claim 7, **characterized in that** the return means is a spring (5) bearing against said cross-member (14).

9. A device according to claim 2 and any one of claims 3 to 8, **characterized in that** said case (13) is constituted by s,parable portions to give access to the fixing zone between the top piston portion (3B) and the bottom piston portion (3A).

10. A device according to any one of claims 2 to 9, **characterized in that** said case (13) is closed at its end remote from the chamber by a cover (20) which allows manual thrust to be applied to the piston but which prevents, unless broken, the portion of the piston that includes said reservoir (7) being extracted from the case.

11. A device according to any one of claims 1 to 10, **characterized in that** the portion of the piston (3) which constitutes the reservoir (7) contains a plug (17) which follows the level of the liquid in the reservoir, preventing any empty space existing above the liquid in the reservoir, and guaranteeing that the device is leakproof in any position.

12. A device according to claim 11, **characterized in that** it has transparent or pierced portions so that the plug (17) is visible from outside the device so as to indicate the volume of liquid remaining in the reservoir.

13. A device according to any one of claims 1 to 12, **characterized in that** said flow rate limiter (9) is a capillary tube that is selected as a function of the reference value.
